# EUROPEAN PATENT SPECIFICATION

(11) **EP 4 424 243 B1**
(45) Date of publication and mention of the grant of the patent: **03.06.2026**
(21) Application number: 23158915.1
(22) Date of filing: 28.02.2023
(51) Int. Cl.: A61B 10/00, A61B 10/04

(54) **DEVICE AND METHOD FOR COLLECTING AND CLEANING SOLID SUBSTANCES**
VORRICHTUNG UND VERFAHREN ZUM SAMMELN UND REINIGEN VON FESTSTOFFEN
DISPOSITIF ET PROCÉDÉ DE COLLECTE ET DE NETTOYAGE DE SUBSTANCES SOLIDES

(43) Date of publication of application: 04.09.2024
(73) Proprietor: Stichting IMEC Nederland, 6708 WH Wageningen (NL)
(72) Inventor: Schoeman, Rogier, 6708 RD Wageningen (NL); Mc Sharry, Neil, 6708 HP Wageningen (NL)
(74) Representative: Roth, Sebastian

(56) References cited:
- CN-A- 102 370 447
- CN-U- 207 908 510
- CN-U- 210 080 214
- CN-U- 215 189 273
- US-A1- 2012 101 481
- US-A1- 2019 307 434

## Description

The invention relates to the collection and cleaning of solid substances, especially to provide a convenient means to retrieve and to clean ingestible sampling pills after excretion so that the contents of the capsule can be collected and tested.

Generally, removing as much faeces as possible from the exterior of ingestible sampling pills, especially retrieved after excretion, is of paramount importance for the retrieval process and user experience. The process of cleaning the pill and the process of separating it from the faeces collectively reduce the risk of contamination of the sample during transport, e.g., to a testing facility. This process further improves the sanitation for those handling the pill and reduces any aversion to contact with faeces from the user retrieving it.

For example, the device "CapsoRetrieve Collector Pan", developed by CapsoVision Inc. (see https://capsovision.com/international/capsocam-accessories/), aims to solve the problem of the retrieval of sensing pills after excretion. The device facilitates a collection pan to be positioned in the toilet and after excretion, the cleaning of the retrieved pill is done by pouring water onto the pan, e.g., by means of a toilet flush. A toilet flush may offer sufficient cleaning power to remove faeces on the higher end of the Bristol Stool Chart, i.e., stool which is runny, with a high-water content.

However, the cleaning power may not be sufficient to separate the pill from more solid faecal matter. Moreover, a pill embedded within a more viscous stool may not be liberated by a single flush and may require several flushes to achieve the preferred level of cleanliness. Multiple toilet flushes not only waste a considerable quantity of water but also time consuming and generally inefficient method.

Chinese patent publication CN 210080214 U discloses a device according to the preamble of claim 1.

Accordingly, an object of the invention is to provide a device and a method for retrieving and cleaning solid substances, e.g., for retrieving ingestible sampling pills after excretion by collecting and cleaning the ingestible sampling pills to separate from the faeces, in a fast and efficient manner.

The object is solved by the features of the first independent claim for the device and by the features of the second independent claim for the method. The dependent claims contain further developments.

According to a first aspect of the invention, a device is provided as defined in claim 1.

In this regard, the fitting arrangement is configured to simultaneously rotate the second slotted container within the first slotted container in a direction opposite to the rotational direction of the driving shaft, especially based on the rotational movement of the driving shaft.

Preferably, the second slotted container is configured to hold the solid body. In other words, the solid body is to be collected or to be placed in the second slotted container, especially during the cleaning phase. The counter rotations of the second slotted container and the driving shaft may generate counter rotational forces, i.e., generating the turbulence and shear force, especially around the solid body within the second slotted container.

The counter rotational force or shear force may be caused due to the viscous action of the fluid, e.g., water, around the solid body during the cleaning phase, especially resulted from the counter rotations of the second slotted container and the driving shaft. In this regard, the amount of shear force may be defined as the unit area amount of force that may act on the fluid parallel to the surface of the solid body.

Therefore, the combined cleaning force generated by the rotating parts, i.e., the centrifugal force generated through the rotation of the second slotted container and the turbulence and shear force generated through the opposing rotations of the second slotted container and the driving shaft, may provide a low impact means of cleaning to remove the dirt particles from the solid body in a fast and efficient manner, whereby reducing the risk of damage to the solid body surface.

Preferably, the driving shaft comprises a first member comprising a first end and a second end, and a second member attached to the first end of the first member via a further fitting arrangement. In this regard, the second end of the first member is configured to be detachably attached to the fitting arrangement.

Advantageously, the container arrangement can be placed at a suitable place such as at the narrow bottom section of a toilet and/or in a bucket, whereby the driving shaft may be detached during the collection phase, e.g., during excretion, and may be attached again during the cleaning and/or retrieving phase.

Furthermore, the second member is configured to move along the first member in a lateral movement direction. Moreover, the further fitting arrangement is configured to rotate the first member along the rotational direction based on the lateral movement of the second member.

Advantageously, the linear to rotational motion conversion, i.e., the conversion of the linear motion of the second member into a one-way rotational motion of the first member, facilitates a comfortable and ease of cleaning operation, especially to rotate the second slotted container via the driving shaft.

Preferably, the first member of the driving shaft further comprises a brush arrangement, especially at or near the second end. More preferably, the first member of the driving shaft, especially the brush arrangement, may comprise a plurality of bristles at or near the second end. Advantageously, the brush arrangement may also contribute to the turbulent flow and may further facilitate a more aggressive cleaning to remove the dirt particles from the solid body.

Additionally or alternatively, the first member of the driving shaft further comprises a plurality of lateral fins, especially at or near the second end. Advantageously, the fins may further increase the turbulent flow, thereby increasing the low impact cleaning power.

Preferably, the first member of the driving shaft further comprises a first set of interlocking teeth at the first end. In addition, the further fitting arrangement comprises a rotatable member comprising a second set of interlocking teeth configured to interlock with the first set of interlocking teeth of the first member. Furthermore, the rotatable member comprises longitudinal helical grooves.

Moreover, the second member of the driving shaft comprises a plurality of internal spheres configured to interlock with the longitudinal helical grooves of the rotatable member. Advantageously, the linear motion of the second member can be converted into a one-way rotational motion of the first member in a simplified manner.

Preferably, the fitting arrangement comprises a first fastening member configured to attach the driving shaft to the fitting arrangement, whereby the first fastening member comprises a third set of interlocking teeth. In addition, the fitting arrangement comprises a second fastening member comprising a fourth set of interlocking teeth configured to interlock with the third set of interlocking teeth of the first fastening member.

Furthermore, the second slotted container comprises a fifth set of interlocking teeth configured to interlock with the fourth set of interlocking teeth of the second fastening member. Advantageously, the one-way rotational motion of the driving shaft can be converted into a one-way rotational motion of the second slotted container, especially in the opposite direction, in a simplified manner.

Preferably, the container arrangement further comprises a ball bearing assembly between the first slotted container and the second slotted container. This may advantageously facilitate the independent rotation of the second slotted container with respect to the first slotted container, especially within the first slotted container.

Preferably, the first slotted container and the second slotted container each comprises a plurality of symmetrical or asymmetrical slots. Advantageously, the respective slots and the centrifugal force generated by the second slotted container may effectively carry away the dirt particles through the slots with ease.

Preferably, the first slotted container comprises a plurality of slots with a first slot dimension and the second slotted container comprises a plurality of slots with a second slot dimension, whereby the first slot dimension is greater than the second slot dimension. For example, the second slotted container may comprise slots with a slot dimension of about 7 mm X 38 mm. Advantageously, the second slotted container may experience a greater exposure, which may further improve the cleaning process.

Preferably, the container arrangement is configured to be partially or fully submerged in a cleansing fluid, e.g., water. For example, the container arrangement may be placed at the narrow bottom section of a toilet or in an external water bucket. This may allow all the water from a toilet flush or in the bucket to be concentrated into one area, thereby facilitating more of the water to directly contact and clean the solid body.

Moreover, the concentration of water at the narrow bottom section of the toilet, especially combined with the added depth and the force of the oncoming water, may result in higher pressure and hence a faster flow of the water over the solid body, which may result in a greater cleaning force, thereby improving the water capacity for carrying away the dirt particles.

Preferably, the container arrangement, especially the first slotted container and/or the second slotted container, comprises or is coated with a hydrophobic material or a superhydrophobic material such as polylactic acid (PLA), polyethylene (PE), high-density polyethylene (HDPE), polyetheretherketone (PEEK), poly-methyl methacrylate (PMMA), manganese-oxide polystyrene (MnO2/PS), zinc-oxide polystyrene (ZnO/PS), precipitated calcium carbonate (PCC), and so on.

Additionally, the driving shaft, especially the first member and/or the brush arrangement and/or the fins of the driving shaft, may comprise or be coated with a hydrophobic material or a superhydrophobic material.

Preferably, the solid body is an ingestible sampling pill. Additionally or alternatively, the solid body may be one of the commercially available capsule endoscopy products. Further alternatively, the solid body may correspond to any swallowed or anally lodged solid substances.

According to a second aspect of the invention, a method is provided for cleaning dirt particles from a solid body, as defined in claim 13.

Preferably, the method further comprises the steps of providing the driving shaft by attaching a second member to a first end of a first member via a further fitting arrangement and further by detachably attaching a second end of the first member to the fitting arrangement, moving the second member along the first member in a lateral movement direction, and rotating the first member by means of the further fitting arrangement along the rotational direction based on the lateral movement of the second member.

Preferably, the method further comprises the steps of containing or holding the solid body by the second slotted container, and generating a counter rotational force or shear force at or around the solid body by simultaneously rotating the second slotted container in the direction opposite to the rotational direction of the driving shaft, i.e., by the opposite rotations of the driving shaft and the second slotted container, especially for cleaning the dirt particles on the solid body.

It is to be noted that the method according to the second aspect corresponds to the device according to the first aspect and its implementation forms. Accordingly, the method of the second aspect may have corresponding implementation forms. Further, the method of the second aspect achieves the same advantages and effects as the device of the first aspect and its respective implementation forms.

Exemplary embodiments of the invention are now further explained with respect to the drawings by way of example only, and not for limitation. In the drawings:
Fig. 1 shows an exemplary embodiment of the device;
Fig. 2A-2D show a first exemplary embodiment of the driving shaft;
Fig. 3A shows a second exemplary embodiment of the driving shaft;
Fig. 3B shows a third exemplary embodiment of the driving shaft;
Fig. 4A-4C show a first exemplary embodiment of the container arrangement;
Fig. 5A shows the fitting arrangement in detail;
Fig. 5B shows a second exemplary embodiment of the container arrangement; and
Fig. 6 shows an exemplary embodiment of the method.

Reference will now be made in detail to exemplary embodiments of the present disclosure, which are illustrated in the accompanying drawings. However, the following embodiments of the present disclosure may be variously modified and the range of the disclosure is not limited by the following embodiments. It is to be noted that the drawings are not scaled relative to each other. Moreover, reference signs for similar entities in different embodiments are partially omitted.

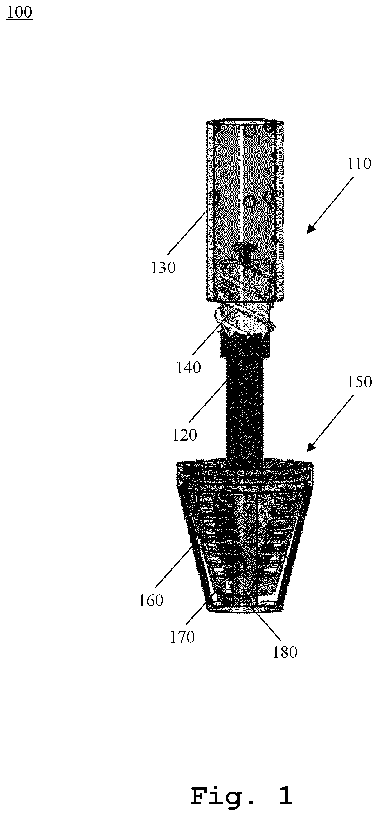

In Fig. 1, an exemplary embodiment of the device 100 according to the first aspect of the invention is illustrated. Particularly, the device 100 is provided for retrieving ingestible sampling pills after excretion, especially by collecting and cleaning the ingestible sampling pills to separate from the faeces.

The device 100 comprises a driving shaft 110 that may further comprise a first member 120 or a shaft base, a second member 130 or a handle, and a fitting arrangement 140 or a rotating part. In this regard, the second member 130 may be moved along the first member 120, especially over the fitting arrangement 140, whereby the fitting arrangement 140 may convert the linear motion of the second member 130 into one-way rotational motion, thereby rotating the first member 120 in a rotational direction.

The device 100 further comprises a container arrangement 150 that comprises a first slotted container 160 or a base container, a second slotted container 170 or a rotating container, and a fitting arrangement 180 or a gear arrangement. In this regard, the second slotted container 170 is attached to the first slotted container 160 and further to the driving shaft 110, especially to the first member 120 of the driving shaft 110 via the fitting arrangement 180.

Moreover, the fitting arrangement 180 causes the second slotted container 170 to rotate, especially by transferring the one-way rotational motion of the driving shaft 110 to the second slotted container 170. Accordingly, the fitting arrangement 180 rotates the second slotted container 170 within the first slotted container 160 in a direction opposite to the rotational direction of the driving shaft 110, especially to the rotational direction of the first member 120 of the driving shaft 110.

The driving shaft 110, especially the first member 120 of the driving shaft 110, may be detachably attached to the fitting arrangement 180 within the container arrangement 150. In other words, the driving shaft 110 and the container arrangement 150 may be separable.

The container arrangement 150 may comprise a conical shape, especially a flat-conical shape flattened at the bottom end of the container arrangement 150. Alternatively, the container arrangement 150 may comprise a taperedcylindrical shape. Further alternatively, the container arrangement 150 may comprise a cylindrical shape. The second slotted container 170 may have a smaller dimension compared to the first slotted container 160 in order to facilitate the rotation of the second slotted container 170 within the first slotted container 160.

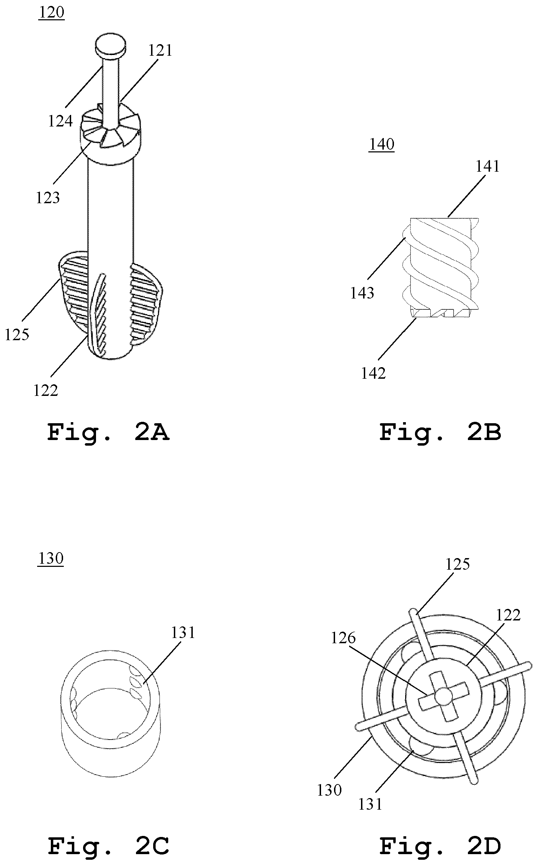

Along Figs. 2A-2D, a first exemplary embodiment of the driving shaft 110 is illustrated. In particular, Fig. 2A shows the first member 120 of the driving shaft 110 in detail. The first member 120 may comprise a first end 121 and a second end 122. The first end 121 may comprise a set of interlocking teeth 123. The first end 121 may further comprise a thinned region 124, especially to position or to affix the fitting arrangement 140 at the first end 121 of the first member 120.

As such, the first member 120 may comprise a solid cylindrical shape with the thinned region 124 and the set of interlocking teeth 123 facilitated at the first end 121. Furthermore, the second end 122 may comprise a plurality of fins or fan blades 125, especially a plurality of fins laterally affixed along the second end 122 of the first member 120. The fins 125 may comprise or be slotted fins and/or solid fins.

The fins 125 may comprise a rectangular shape, a swept shape, a tapered swept shape, a clipped delta shape, a trapezoidal shape, an elliptical shape, or the like. The fins 125 may be formed integrally with respect to the first member 120, i.e., comprising a material homogeneous to the material of the first member 120.

Alternatively, the fins 125 may comprise a heterogeneous material and may be affixed to the first member 120 at the second end 122 via glue or screws or other fastening means. The second end 122 may further comprise recesses 126, which is shown in Fig. 2D, in order to attach the first member 120 with the fitting arrangement 180 of the container arrangement 150.

Fig. 2B shows the fitting arrangement 140 of the driving shaft 110 in detail. The fitting arrangement 140 may comprise or be a rotatable member 141 comprising a hollowed-cylindrical shape with a set of interlocking teeth 142, especially formed at one end of the rotatable member 141, and longitudinal helical grooves 143, especially formed at the outer surface of the rotatable member 141.

For example, the set of interlocking teeth 142 may be formed to interlock with the set of interlocking teeth 123 at the first end 121 of the first member 120. Furthermore, the hollowed inner section of the rotatable member 141 may be formed to match the thinned region 124 at the first end 121 of the first member 120, especially to affix the rotatable member 141 at the first end 121 of the first member 120.

The fitting arrangement 140, especially the rotatable member 141, may be formed integrally with respect to the first member 120, i.e., comprising a material homogeneous to the material of the first member 120. Alternatively, the fitting arrangement 140, especially the rotatable member 141, may comprise a homogeneous or a heterogeneous material and may be affixed to the first member 120 at the first end 121 via glue or screws or other fastening means.

Fig. 2C shows the second member 130 of the driving shaft 110 in detail. The second member 130 may comprise a hollowed-cylindrical shape with a plurality of internal spheres 131, especially formed at the inner surface of the second member 130.

The hollowed-cylindrical shape of the second member 130 may be formed so as to encompass the fitting arrangement 140, especially the rotatable member 141, at the first end 121 of the first member 120 while moving along the first member 120 over the rotatable member 141 in the lateral movement direction. The plurality of internal spheres 131 of the second member 130 may be formed to interlock with the longitudinal helical grooves 143 of the rotatable member 141, especially while moving in the lateral movement direction.

Fig. 2D shows a bottom view of the first exemplary embodiment of the driving shaft 110. It can be seen that the first member 120, especially the bottom edge at the second end 122, may comprise recesses 126, e.g., cross-shaped recesses as shown herein, in order to attach the first member 120 with the fitting arrangement 180 of the container arrangement 150 in a detachable manner.

Therefore, the second member 130 may be freely moved in the lateral movement direction over the rotatable member 141 along the first member 120. The interlocking between the plurality of internal spheres 131 of the second member 130 and the longitudinal helical grooves 143 of the rotatable member 141 may cause the rotatable member 141 to rotate in the rotational direction, thereby translating or converting the lateral movement of the second member 130 into one-way rotational movement of the rotatable member 141.

Furthermore, the interlocking between the set of interlocking teeth 123 of the first member 120 and the set of interlocking teeth 142 of the rotatable member 141 may cause the first member 120 to rotate in the rotational direction, thereby transferring the one-way rotational movement of the rotatable member 141 to the first member 120.

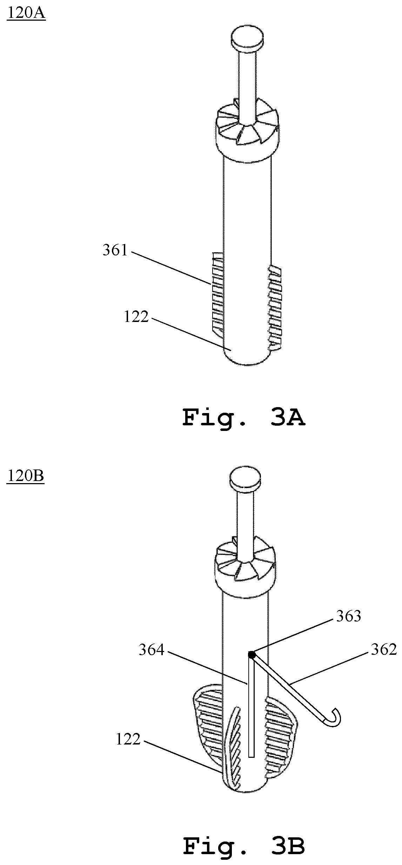

Fig. 3A shows a second exemplary embodiment of the driving shaft 110. In particular, a second exemplary embodiment of the first member 120A of the driving shaft 110 is illustrated herein. The first member 120A differs from the first member 120 in that the first member 120A may comprise a brush arrangement 361 at the second end 122 of the first member 120A, especially in addition to or as an alternative to the plurality of fins 125. The brush arrangement 361 may comprise a plurality of bristles comprising or be made of synthetic fiber, e.g., polyamide, polyester, polypropylene, and so on.

Fig. 3B shows a third exemplary embodiment of the driving shaft 110. In particular, a third exemplary embodiment of the first member 120B of the driving shaft 110 is illustrated herein. The first member 120B differs from the first member 120 in that the first member 120B may comprise a hook arrangement 362 in order to facilitate the lifting of the container arrangement 150 for retrieving the pills after the cleaning phase and/or to re-position the container arrangement 150 to a different location and/or setup.

In this regard, the hook arrangement 362 may comprise a hook with an elongated end, which may be attached to the first member 120B via screws or springs 363. The first member 120B may further comprise a shallow groove 364, especially at the outer surface, in order to fully accommodate and thereby encompassing the hook arrangement 362.

For example, during the cleaning phase, i.e., when the first member 120B is rotated, the hook arrangement 362 may reside within the shallow groove 364. Furthermore, after the cleaning phase, i.e., when the first member 120B is standstill, the hook arrangement 362 may be ejected or be sprung from the shallow groove 364, e.g., either manually or via the spring 363.

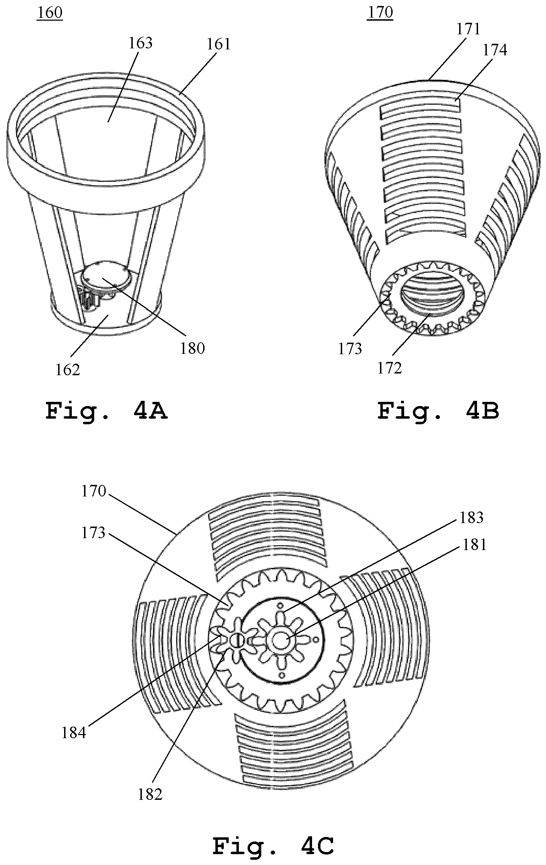

Along Figs. 4A-4C, a first exemplary embodiment of the container arrangement 150 is illustrated. In particular, Fig. 4A shows the first slotted container 160 of the container arrangement 150 in detail. The first slotted container 160 may comprise or be a hollowed-tapered cylindrical container comprising a hollowed top part 161 and a solid bottom part or base 162. The first slotted container 160 may further comprise a plurality of slots 163, especially comprising slots tapered from the top part 161 to the bottom part 162. The first slotted container 160 may further comprise the fitting arrangement 180, especially at the solid bottom part 162.

Fig. 4B shows the second slotted container 170 of the container arrangement 150 in detail. The second slotted container 170 may comprise or be a hollowed-tapered cylindrical container comprising a hollowed top part 171 and a hollowed bottom part or base 172. The second slotted container 170 may further comprise a set of interlocking teeth 173, especially at the bottom part 172.

The second slotted container 170 may further comprise a plurality of slots 174, especially comprising slots formed along the tapered surface from the top part 171 to the bottom part 172. For example, each of the plurality of slots 174 may comprise a slot dimension of about 7 mm X 38 mm.

Fig. 4C shows the fitting arrangement 180 of the container arrangement 150. The fitting arrangement 180 may comprise a first fastening member or gear 181 comprising a set of interlocking teeth 183. The fitting arrangement 180 may further comprise a second fastening member or gear 182 comprising a set of interlocking teeth 184.

In this regard, the set of interlocking teeth 184 of the second fastening member 182 may be formed to interlock with the set of interlocking teeth 183 of the first fastening member 181. Furthermore, the set of interlocking teeth 184 of the second fastening member 182 may be formed to interlock with the set of interlocking teeth 173 of the second slotted container 170, e.g., at the bottom part 172.

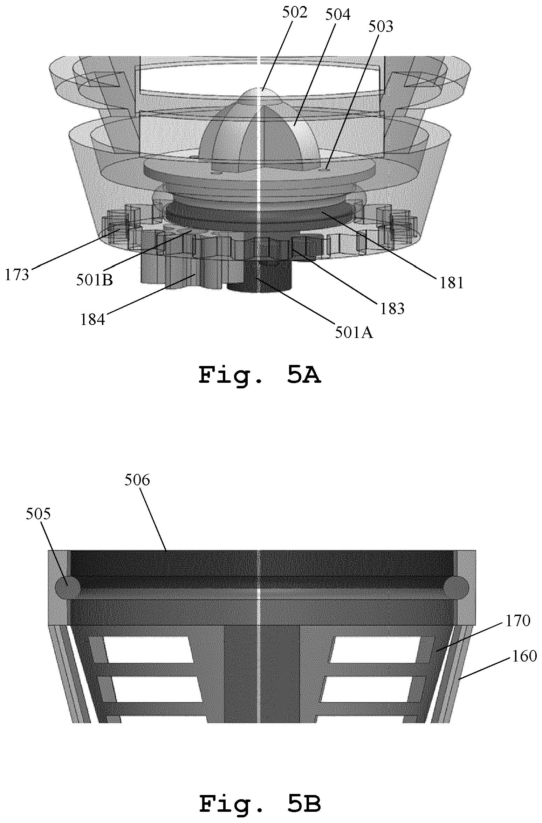

Fig. 5A further shows the fitting arrangement 180 of the container arrangement 150 in detail. The fitting arrangement 180 may further comprise a first base 501A and a second base 501B. The first base 501A and the second base 501B may be fixed at the solid bottom part 162 of the first slotted container 160.

In this regard, the first base 501A may be formed to affix the first fastening member 181 in a rotatable manner, i.e., to facilitate a free rotation of the first fastening member 181 at the first base 501A. Furthermore, the second base 501B may be formed to affix the second fastening member 182 in a rotatable manner, i.e., to facilitate a free rotation of the second fastening member 182 at the second base 501B.

Moreover, the fitting arrangement 180, especially the first fastening member 181, may comprise a third fastening member 502, preferably attached to the first fastening member 181. The third fastening member 502 may comprise a flat base 504 and a set of interlocking slots 503. The flat base 504 may be formed to affix the third fastening member 502 on top of the first fastening member 181, **e.g.,** via glue or screws.

Alternatively, the third fastening member 502 may be integrally formed with the first fastening member 181. Furthermore, the set of interlocking slots 503 may be formed to interlock with the recesses 126 of the first member 120 of the driving shaft 110, especially at the second end 122 of the first member 120 of the driving shaft 110.

It is to be noted that the hollowed bottom part 172 of the second slotted container 170 may comprise an inner-diameter such that the third fastening member 502 may be passed through and be able to attach the driving shaft 110 accordingly.

Therefore, the rotational movement of the driving shaft 110, especially of the first member 120 of the driving shaft 110, along the rotational direction may be transferred to the first fastening member 181 via the third fastening member 502. Furthermore, the interlocking between the set of interlocking teeth 183 of the first fastening member 181 and the set of interlocking teeth 184 of the second fastening member 182 may cause the second fastening member 182 to rotate in a direction opposite to the rotational direction of the first fastening member 181.

Moreover, the set of interlocking teeth 184 of the second fastening member 182 and the set of interlocking teeth 173 of the second slotted container 170 may cause the second slotted container 170 to rotate in the rotational direction of the second fastening member 182.

Accordingly, the fitting arrangement 180 of the container arrangement 150 may translate the rotational movement of the driving shaft 110 to the container arrangement 150, especially to rotate the second slotted container 170 within the first slotted container 160 in the direction opposite to the rotational direction of the driving shaft 110.

Fig. 5B shows a second exemplary embodiment of the container arrangement 150. The container arrangement 150 illustrated herein may additionally comprise a ball-bearing arrangement or assembly 505 between the first slotted container 160 and the second slotted container 170. For example, the hollowed top part 161 of the first slotted container 160 and the hollowed top part 171 of the second slotted container 170 may be formed, e.g., with grooves or channels, to comprise the ball-bearing 505 between the respective top parts.

Furthermore, the container arrangement 150 may further comprise a cap 506 to encompass the respective top parts of the container arrangement 150 as well as to cover the ball-bearing 505, whereby allowing the free rotation of the second slotted container 170 within the first slotted container 160. The cap 506 may additionally provide protection against faeces and/or toilet paper from interfering with the ball-bearing 505.

Additionally or alternatively, the container arrangement 150 may comprise a further ball-bearing (not shown) between the hollowed bottom part 172 of the second slotted container 170 and the first fastening member 181/the third fastening member 502, especially to enhance the free rotation of the second slotted container 170.

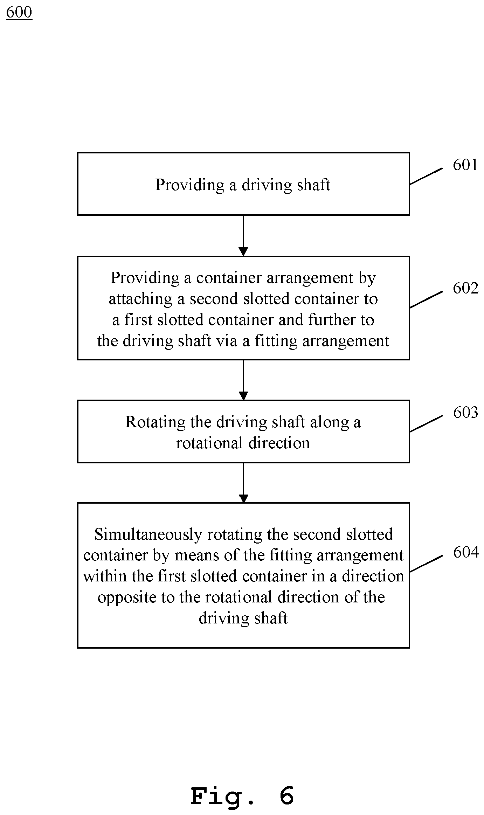

In Fig. 6, an exemplary embodiment of the method 600 according to the second aspect of the invention is illustrated. In a first step 601, a driving shaft is provided, the driving shaft being configured to rotate along a rotational direction. In a second step 602, a container arrangement is provided by attaching a second slotted container to a first slotted container and further to the driving shaft via a fitting arrangement.

In a third step 603, the driving shaft is rotated along the rotational direction. In a fourth step 604, the second slotted container is simultaneously rotated by means of the fitting arrangement within the first slotted container in a direction opposite to the rotational direction of the driving shaft.

Therefore, the proposed solution utilizes a combination of turbulence and shear force, centrifugal force, and mechanical process to clean sensing pills, especially retrieved after excretion. The turbulence and shear may be generated through the opposing rotations of the rotating container and the driving shaft and optionally of the fins and/or the brushes attached to the driving shaft. Use of said turbulence and shear force may provide a low impact means of cleaning, thereby reducing the risk of damage to the pill.

Moreover, the brush arrangement, while also may contribute to the turbulent flow, may provide a slightly more aggressive cleaning component to aid in the breakup of larger or tougher pieces of faeces while posing minimum risk to the pill's integrity. The spinning motion of the rotating container may impart a centrifugal force on the faecal fragments contained, which may in turn pull them to the sides of the rotating container and through the slots. The slots, especially the slots of the rotating container, may be advantageously sized so as not to allow the pill itself to escape but only to remove the pieces of faeces.

It is to be noted that human faeces may experience a thinning behavior with shear rates as low 1s⁻¹. Furthermore, at shear rates of 10s⁻¹ for 20 seconds, the viscosity of the faeces may decrease to 0.5% of its original value. The embodiments of the proposed solution may generate a shear rate of at least one to two orders of magnitude higher than the above value.

It is important to note that, in the description as well as in the claims, the word "comprising" does not exclude other elements or steps and the indefinite article "a" or "an" does not exclude a plurality. A single element or other unit may fulfill the functions of several entities or items recited in the claims. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A device (100) for cleaning dirt particles from a solid body comprising:
a driving shaft (110) configured to rotate along a rotational direction, and
a container arrangement (150) comprising a first slotted container (160), and **characterised by** further comprising a second slotted container (170) attached to the first slotted container (160) and further to the driving shaft (110) within the container arrangement (150) via a fitting arrangement (180),
wherein the fitting arrangement (180) is configured to translate the rotational movement of the driving shaft (110) to rotate the second slotted container (170) within the first slotted container (160) in a direction opposite to the rotational direction of the driving shaft (110).

2. The device according to claim 1,
wherein the driving shaft (110) comprises:
a first member (120) comprising a first end (121) and a second end (122), and
a second member (130) attached to the first end (121) of the first member (120) via a further fitting arrangement (140),
wherein the second end (122) of the first member (120) is configured to be detachably attached to the fitting arrangement (180),
wherein the second member (130) is configured to move along the first member (120) in a lateral movement direction, and wherein the further fitting arrangement (140) is configured to rotate the first member (120) along the rotational direction based on the lateral movement of the second member (130).

3. The device according to claim 2,
wherein the first member (120) of the driving shaft (110) further comprises a brush arrangement (361), especially at or near the second end (122), and/or
wherein the first member (120) of the driving shaft (110) further comprises a plurality of lateral fins (125), especially at or near the second end (122).

4. The device according to claim 2 or 3,
wherein the first member (120) further comprises a first set of interlocking teeth (123) at the first end (121), and wherein the further fitting arrangement (140) comprises a rotatable member (141) comprising a second set of interlocking teeth (142) configured to interlock with the first set of interlocking teeth (123) of the first member (120).

5. The device according to claim 4,
wherein the rotatable member (141) further comprises longitudinal helical grooves (143), and
wherein the second member (130) comprises a plurality of internal spheres (131) configured to interlock with the longitudinal helical grooves (143) of the rotatable member (141).

6. The device according to any of claims 1 to 5,
wherein the fitting arrangement (180) comprises:
a first fastening member (181) configured to attach the driving shaft (110) to the fitting arrangement (180), whereby the first fastening member (181) comprises a third set of interlocking teeth (183), and
a second fastening member (182) comprising a fourth set of interlocking teeth (184) configured to interlock with the third set of interlocking teeth (183) of the first fastening member (181).

7. The device according to claim 6,
wherein the second slotted container (170) comprises a fifth set of interlocking teeth (173) configured to interlock with the fourth set of interlocking teeth (184) of the second fastening member (182).

8. The device according to any of claims 1 to 7,
wherein the container arrangement (150) further comprises a ball bearing assembly (505) between the first slotted container (160) and the second slotted container (170).

9. The device according to any of claims 1 to 8,
wherein the first slotted container (160) and the second slotted container (170) each comprises a plurality of symmetrical or asymmetrical slots.

10. The device according to any of claims 1 to 9,
wherein the first slotted container (160) comprises a plurality of slots (163) with a first slot dimension and the second slotted container (170) comprises a plurality of slots (174) with a second slot dimension, whereby the first slot dimension is greater than the second slot dimension.

11. The device according to any of claims 1 to 10,
wherein the container arrangement (150) is configured to be partially or fully submerged in a cleansing fluid, and/or wherein the solid body is an ingestible sampling pill.

12. The device according to any of claims 1 to 11,
wherein the container arrangement (150), especially the first slotted container (160) and/or the second slotted container (170), comprises or is coated with a hydrophobic material or a superhydrophobic material.

13. A method (600) for using the device (100) according to any of claims 1 to 12 comprising:
rotating (603) the driving shaft within the container arrangement along the rotational direction, and
rotating (604) the second slotted container by translating the rotational movement of the driving shaft by means of the fitting arrangement within the first slotted container in a direction opposite to the rotational direction of the driving shaft.

14. The method according to claim 13, further comprising:
providing the driving shaft by attaching a second member to a first end of a first member via a further fitting arrangement and further by detachably attaching a second end of the first member to the fitting arrangement,
moving the second member along the first member in a lateral movement direction, and
rotating the first member by means of the further fitting arrangement along the rotational direction based on the lateral movement of the second member.

15. The method according to claim 13 or 14, further comprising:
containing the solid body by the second slotted container, and
generating a counter rotational force at or around the solid body by simultaneously rotating the second slotted container in the direction opposite to the rotational direction of the driving shaft, especially for cleaning the dirt particles on the solid body.

## Patentansprüche

1. Vorrichtung (100) zum Reinigen von Schmutzpartikeln von einem Festkörper, umfassend:
eine Antriebswelle (110), die dazu ausgelegt ist, sich entlang einer Drehrichtung zu drehen, und
eine Behälteranordnung (150), die einen ersten geschlitzten Behälter (160) umfasst, und **dadurch gekennzeichnet, dass** sie ferner einen zweiten geschlitzten Behälter (170) umfasst, der an dem ersten geschlitzten Behälter (160) befestigt ist und ferner innerhalb der Behälteranordnung (150) über eine Verbindungsanordnung (180) an der Antriebswelle (110) befestigt ist,
wobei die Verbindungsanordnung (180) dazu ausgelegt ist, die Drehbewegung der Antriebswelle (110) umzusetzen, um den zweiten geschlitzten Behälter (170) innerhalb des ersten geschlitzten Behälters (160) in einer der Drehrichtung der Antriebswelle (110) entgegengesetzten Richtung zu drehen.

2. Vorrichtung nach Anspruch 1,
wobei die Antriebswelle (110) umfasst:
ein erstes Element (120), umfassend ein erstes Ende (121) und ein zweites Ende (122), und
ein zweites Element (130), das über eine weitere Verbindungsanordnung (140) am ersten Ende (121) des ersten Elements (120) befestigt ist,
wobei das zweite Ende (122) des ersten Elements (120) dazu ausgelegt ist, lösbar an der Befestigungsanordnung (180) befestigt zu werden,
wobei das zweite Element (130) dazu ausgelegt ist, sich entlang des ersten Elements (120) in einer lateralen Bewegungsrichtung zu bewegen, und wobei die weitere Verbindungsanordnung (140) dazu ausgelegt ist, das erste Element (120) basierend auf der lateralen Bewegung des zweiten Elements (130) in Drehrichtung zu drehen.

3. Vorrichtung nach Anspruch 2,
wobei das erste Element (120) der Antriebswelle (110) ferner eine Bürstenanordnung (361), insbesondere an oder nahe dem zweiten Ende (122), umfasst und/oder
wobei das erste Element (120) der Antriebswelle (110) ferner mehrere seitliche Rippen (125) umfasst, insbesondere an oder nahe dem zweiten Ende (122).

4. Vorrichtung nach Anspruch 2 oder 3,
wobei das erste Element (120) ferner einen ersten Satz von ineinandergreifenden Zähnen (123) am ersten Ende (121) umfasst, und wobei die weitere Verbindungsanordnung (140) ein drehbares Element (141) umfasst, das einen zweiten Satz von ineinandergreifenden Zähnen (142) umfasst, die dazu ausgelegt sind, mit dem ersten Satz von ineinandergreifenden Zähnen (123) des ersten Elements (120) ineinanderzugreifen.

5. Vorrichtung nach Anspruch 4,
wobei das drehbare Element (141) ferner längsverlaufende schraubenförmige Nuten (143) umfasst, und
wobei das zweite Element (130) mehrere innere Kugeln (131) umfasst, die dazu ausgelegt sind, mit den längsverlaufenden schraubenförmigen Nuten (143) des drehbaren Elements (141) ineinanderzugreifen.

6. Vorrichtung nach einem der Ansprüche 1 bis 5,
wobei die Verbindungsanordnung (180) umfasst:
ein erstes Befestigungselement (181), das dazu ausgelegt ist, die Antriebswelle (110) an der Verbindungsanordnung (180) zu befestigen, wobei das erste Befestigungselement (181) einen dritten Satz von ineinandergreifenden Zähnen (183) umfasst, und
ein zweites Befestigungselement (182), das einen vierten Satz von ineinandergreifenden Zähnen (184) umfasst, die dazu ausgelegt sind, mit dem dritten Satz von ineinandergreifenden Zähnen (183) des ersten Befestigungselements (181) ineinanderzugreifen.

7. Vorrichtung nach Anspruch 6,
wobei der zweite geschlitzte Behälter (170) einen fünften Satz von ineinandergreifenden Zähnen (173) umfasst, die dazu ausgelegt sind, mit dem vierten Satz von ineinandergreifenden Zähnen (184) des zweiten Befestigungselements (182) ineinanderzugreifen.

8. Vorrichtung nach einem der Ansprüche 1 bis 7,
wobei die Behälteranordnung (150) ferner eine Kugellageranordnung (505) zwischen dem ersten geschlitzten Behälter (160) und dem zweiten geschlitzten Behälter (170) umfasst.

9. Vorrichtung nach einem der Ansprüche 1 bis 8,
wobei der erste geschlitzte Behälter (160) und der zweite geschlitzte Behälter (170) jeweils mehrere symmetrische oder asymmetrische Schlitze umfassen.

10. Vorrichtung nach einem der Ansprüche 1 bis 9,
wobei der erste geschlitzte Behälter (160) mehrere Schlitze (163) mit einer ersten Schlitzabmessung umfasst und der zweite geschlitzte Behälter (170) mehrere Schlitze (174) mit einer zweiten Schlitzabmessung umfasst, wobei die erste Schlitzabmessung größer als die zweite Schlitzabmessung ist.

11. Vorrichtung nach einem der Ansprüche 1 bis 10,
wobei die Behälteranordnung (150) dazu ausgelegt ist, teilweise oder vollständig in ein Reinigungsfluid eingetaucht zu werden, und/oder wobei der Festkörper eine einnehmbare Probenahmepille ist.

12. Vorrichtung nach einem der Ansprüche 1 bis 11,
wobei die Behälteranordnung (150), insbesondere der erste geschlitzte Behälter (160) und/oder der zweite geschlitzte Behälter (170), ein hydrophobes Material oder ein superhydrophobes Material umfasst oder damit beschichtet ist.

13. Verfahren (600) zum Verwenden der Vorrichtung (100) nach einem der Ansprüche 1 bis 12, umfassend:
Drehen (603) der Antriebswelle innerhalb der Behälteranordnung in Drehrichtung, und
Drehen (604) des zweiten geschlitzten Behälters durch Umsetzen der Drehbewegung der Antriebswelle mittels der Verbindungsanordnung innerhalb des ersten geschlitzten Behälters in einer der Drehrichtung der Antriebswelle entgegengesetzten Richtung.

14. Verfahren nach Anspruch 13, ferner umfassend:
Bereitstellen der Antriebswelle durch Befestigen eines zweiten Elements an einem ersten Ende eines ersten Elements über eine weitere Verbindungsanordnung und ferner durch lösbares Befestigen eines zweiten Endes des ersten Elements an der Verbindungsanordnung,
Bewegen des zweiten Elements entlang des ersten Elements in einer lateralen Bewegungsrichtung, und
Drehen des ersten Elements mittels der weiteren Verbindungsanordnung in Drehrichtung basierend auf der seitlichen Bewegung des zweiten Elements.

15. Verfahren nach Anspruch 13 oder 14, ferner umfassend:
Aufnehmen des Festkörpers durch den zweiten geschlitzten Behälter und
Erzeugen einer gegenläufigen Rotationskraft an oder um den Festkörper durch gleichzeitiges Drehen des zweiten geschlitzten Behälters in einer der Drehrichtung der Antriebswelle entgegengesetzten Richtung, insbesondere zum Reinigen der Schmutzpartikel auf dem Festkörper.

## Revendications

1. Dispositif (100) pour le nettoyage d'un corps solide de façon à éliminer des particules de saleté présentes sur celui-ci comprenant :
un arbre d'entraînement (110) conçu pour tourner dans un sens de rotation, et
un système de réceptacle (150) comprenant un premier réceptacle à fentes (160), et **caractérisé en ce qu'**il comprend en outre un second réceptacle à fentes (170) fixé au premier réceptacle à fentes (160) et en outre à l'arbre d'entraînement (110) à l'intérieur du système de réceptacle (150) par l'intermédiaire d'un système de raccord (180),
le système de raccord (180) étant conçu pour convertir le mouvement de rotation de l'arbre d'entraînement (110) afin de faire tourner le second réceptacle à fentes (170) à l'intérieur du premier réceptacle à fentes (160) dans un sens opposé au sens de rotation de l'arbre d'entraînement (110).

2. Dispositif selon la revendication 1,
dans lequel l'arbre d'entraînement (110) comprend :
un premier élément (120) comprenant une première extrémité (121) et une seconde extrémité (122), et
un second élément (130) fixé à la première extrémité (121) du premier élément (120) par l'intermédiaire d'un autre système de raccord (140),
dans lequel la seconde extrémité (122) du premier élément (120) est conçue pour être fixée de manière détachable au système de raccord (180),
dans lequel le second élément (130) est conçu pour se déplacer le long du premier élément (120) dans une direction de mouvement latéral, et dans lequel l'autre système de raccord (140) est conçu pour faire tourner le premier élément (120) dans le sens de rotation sur la base du mouvement latéral du second élément (130).

3. Dispositif selon la revendication 2,
dans lequel le premier élément (120) de l'arbre d'entraînement (110) comprend en outre un système de brosse (361), notamment au niveau de la seconde extrémité (122) ou à proximité de celle-ci, et/ou
dans lequel le premier élément (120) de l'arbre d'entraînement (110) comprend en outre une pluralité d'ailettes latérales (125), notamment au niveau de la seconde extrémité (122) ou à proximité de celle-ci.

4. Dispositif selon la revendication 2 ou 3,
dans lequel le premier élément (120) comprend en outre un premier ensemble de dents d'engrènement (123) au niveau de la première extrémité (121), et dans lequel l'autre système de raccord (140) comprend un élément rotatif (141) comprenant un deuxième ensemble de dents d'engrènement (142) conçues pour s'engrener avec le premier ensemble de dents d'engrènement (123) du premier élément (120).

5. Dispositif selon la revendication 4,
dans lequel l'élément rotatif (141) comprend en outre des rainures hélicoïdales longitudinales (143), et
dans lequel le second élément (130) comprend une pluralité de sphères internes (131) conçues pour coopérer avec les rainures hélicoïdales longitudinales (143) de l'élément rotatif (141).

6. Dispositif selon l'une quelconque des revendications 1 à 5,
dans lequel le système de raccord (180) comprend :
un premier élément de fixation (181) conçu pour fixer l'arbre d'entraînement (110) au système de raccord (180), le premier élément de fixation (181) comprenant un troisième ensemble de dents d'engrènement (183), et
un second élément de fixation (182) comprenant un quatrième ensemble de dents d'engrènement (184) conçues pour s'engrener avec le troisième ensemble de dents d'engrènement (183) du premier élément de fixation (181).

7. Dispositif selon la revendication 6,
dans lequel le second réceptacle à fentes (170) comprend un cinquième ensemble de dents d'engrènement (173) conçues pour s'engrener avec le quatrième ensemble de dents d'engrènement (184) du second élément de fixation (182).

8. Dispositif selon l'une quelconque des revendications 1 à 7,
dans lequel le système de réceptacle (150) comprend en outre un ensemble roulement à billes (505) entre le premier réceptacle à fentes (160) et le second réceptacle à fentes (170).

9. Dispositif selon l'une quelconque des revendications 1 à 8,
dans lequel le premier réceptacle à fentes (160) et le second réceptacle à fentes (170) comprennent chacun une pluralité de fentes symétriques ou asymétriques.

10. Dispositif selon l'une quelconque des revendications 1 à 9,
dans lequel le premier réceptacle à fentes (160) comprend une pluralité de fentes (163) avec une première dimension de fente et le second réceptacle à fentes (170) comprend une pluralité de fentes (174) avec une seconde dimension de fente, la première dimension de fente étant plus grande que la seconde dimension de fente.

11. Dispositif selon l'une quelconque des revendications 1 à 10,
dans lequel le système de réceptacle (150) est conçu pour être partiellement ou totalement immergé dans un fluide de nettoyage, et/ou dans lequel le corps solide est une pilule d'échantillonnage ingérable.

12. Dispositif selon l'une quelconque des revendications 1 à 11,
dans lequel le système de réceptacle (150), en particulier le premier réceptacle à fentes (160) et/ou le second réceptacle à fentes (170), comprend ou est revêtu d'un matériau hydrophobe ou d'un matériau superhydrophobe.

13. Procédé (600) d'utilisation du dispositif (100) selon l'une quelconque des revendications 1 à 12 comprenant :
appliquer une rotation (603) à l'arbre d'entraînement à l'intérieur du système de réceptacle dans le sens de rotation, et
appliquer une rotation (604) au second récipient à fentes par conversion du mouvement de rotation de l'arbre d'entraînement au moyen du système de raccord à l'intérieur du premier réceptacle à fentes dans un sens opposé au sens de rotation de l'arbre d'entraînement.

14. Procédé selon la revendication 13, comprenant en outre :
mettre l'arbre d'entraînement en place en fixant un second élément à une première extrémité d'un premier élément par le biais d'un autre système de raccord et en fixant en outre de manière amovible une seconde extrémité du premier élément au système de raccord,
déplacer le second élément le long du premier élément dans une direction de mouvement latéral, et
appliquer une rotation au premier élément au moyen de l'autre système de raccord dans le sens de rotation sur la base du mouvement latéral du second élément.

15. Procédé selon la revendication 13 ou 14, comprenant en outre :
placer le corps solide dans le second réceptacle à fentes, et
générer une force de contre-rotation au niveau ou autour du corps solide en appliquant simultanément une rotation au second réceptacle à fentes dans le sens opposé au sens de rotation de l'arbre d'entraînement, en particulier pour nettoyer le corps solide de façon à éliminer les particules de saleté présentes sur celui-ci.
